# EUROPEAN PATENT SPECIFICATION

(11) **EP 3 723 479 B1**
(45) Date of publication and mention of the grant of the patent: **22.05.2024**
(21) Application number: 18834080.6
(22) Date of filing: 13.12.2018
(51) Int. Cl.: A01K 67/027, C12N 15/113, C12N 15/89, C12N 15/88

(54) **METHODS FOR THE PRODUCTION OF STERILE FISH**
VERFAHREN ZUR PRODUKTION VON STERILEN FISCHEN
PROCÉDÉS DE PRODUCTION DE POISSON STÉRILE

(30) Priority: 15.12.2017 NO 20172005
(43) Date of publication of application: 21.10.2020
(73) Proprietor: ACD Pharmaceuticals AS, 8370 Leknes (NO)
(72) Inventor: JIMÉNEZ MARTÍNEZ, Juan José, 1513 Moss (NO)
(74) Representative: Hertin und Partner Rechts- und Patentanwälte PartG mbB
(86) International application number: PCT/NO2018/050308
(87) International publication number: WO 2019/117728

(56) References cited:
- WO-A2-2016/187198
- ANDREA PAULI ET AL: "Antisense Oligonucleotide-Mediated Transcript Knockdown in Zebrafish", PLOS ONE, vol. 10, no. 10, 5 October 2015 (2015-10-05), page e0139504, XP055571080, DOI: 10.1371/journal.pone.0139504
- SOIFER HARRIS S ET AL: "Chapter 25: Silencing of Gene Expression by Gymnotic Delivery of Antisense Oligonucleotides", FUNCTIONAL GENOMICS: METHODS AND PROTOCOLS, SECOND EDITION; [METHODS IN MOLECULAR BIOLOGY 1064-3745], HUMANA PRESS, NEW YORK, NY, US, PAGE(S) 333 - 346 , 1 January 2012 (2012-01-01), XP009519393, ISBN: 978-1-61779-423-0 Retrieved from the Internet: URL:http://link.springer.com/10.1007/978-1 -61779-424-7_25 [retrieved on 2011-11-04]

## Description

This invention relates to methods for the production of sterile fish. The methods include disruption of gonadal development in fish through the administration of a gapmer as recited in the claims that lead to the failure of fertile gonadal development, and thus to reproductively sterile fish. The methods are for use in e.g. aquaculture, the aquarium trade or control of invasive species.

Aquaculture is currently one of the fastest growing food-producing industries and is becoming increasingly important to resolve the current and projected global shortfalls in aquatic foods and seafood availability. Optimization of methods and procedures used in aquaculture is increasingly necessary to maximize animal welfare/health and food production and to minimize ecological impact, thereby achieving long-term environmental sustainability of our seafood supplies.

Sterilization, i.e. induced infertility of farmed fish and other egg-producing aquatic animals enhances their growth rate by increasing the conversion of food energy to muscle growth, instead of gonadal development. In addition, if escaped from aquaculture operations to the environment, reproductively sterile farmed fish and other egg-producing aquatic animals, including domesticated, non-native or genetically modified species, will not be able to reproduce or inter-breed with wild populations. This will assist biological containment and prevent genetic contamination of wild populations and/or the establishment in the wild of domestic, non-native or genetically modified farmed fish and other egg-producing aquatic animals.

Sterility in fish and other egg-producing aquatic animals can be achieved by disrupting gonadotropin releasing hormone (GnRH) cell development and/or primordial germ cell (PGC) development, migration and colonization in the gonad of the embryo. This results in failure of gonad development and/or failure of full and proper gonadal functioning, and ultimately the generation of reproductively sterile fish and other egg-producing aquatic animals.

Compounds known to disrupt GnRH cell and/or PGC development, migration and/or survival include antisense oligonucleotides (AON), which inactivate mRNAs essential for GnRH cell and/or PGC formation, migration and/or survival by blocking their translation or otherwise interfere with their processing. Z. Linhartová et al., Theriogenology 84 (2015), 1246-1255 disclose the sterilization of sturgeon by microinjection into sturgeon embryos of antisense morpholino oligonucleotides, which knock-down *dead end* (*dnd*) gene expression. *Dnd* is a vertebrate-specific gene encoding a RNA-binding protein, which is crucial for migration and survival of PGCs.

T. Wong and Y. Zohar, Sci. Rep. 5 (2015), 15822 disclose a bath-immersion method to produce infertile zebrafish which includes the use of Vivo-conjugated morpholino oligonucleotides against zebrafish *dnd.*

WO 2015/073819 A1 and WO 2016/187198 disclose methods for the production of reproductively sterile fish and egg-producing aquatic animals for e.g. aquaculture. Compounds for rendering such animals sterile may be delivered to the eggs of such animals prior to fertilization or water activation of said eggs or post fertilization and water activation by contacting the eggs in an immersion medium including the compounds of interest, and optionally a molecular transporter.

A. Pauli et al., PLOS ONE, vol. 10, no.10, (2015), page e0139504 disclose a method for silencing *dnd* expression in a zebrafish egg, the method comprising introducing a *dnd-*specific gapmer into said egg by microinjection.

Generally, in the antisense technology, mRNA is targeted by Watson-Crick hybridization of a complementary AON. The goal of inhibiting gene expression in a specific way may be accomplished by preventing mRNA maturation, blocking translation or by induction of degradation. To be effective the AON must be able to enter the cell, be stable toward nucleases, be non-toxic and show high binding affinity and specificity toward the target mRNA.

Considerable progress with respect to stability and binding has been made by use of chemically modified AONs. An example of such chemically modified AONs oligonucleotides with a phosphorothioate backbone instead of a phosphodiester backbone or a backbone comprising methylenemorpholine rings (i.e. morpholino oligonucleotides), which enhances resistance to enzymatic degradation. Another example is locked nucleic acids (LNA), a nucleoside comprising a bicyclic sugar moiety comprising a 4'-CH2-O-2' bridge, which "locks" the sugar ring in a conformation that exhibits increased binding affinity towards complementary RNA.

This invention relates to methods as defined in the claims.

In a first embodiment, the invention provides a method for producing sterile fish as defined in claim 1.

Fish include all fish species, including, but not limited to salmonids, e.g. Atlantic salmon, coho salmon, chinook salmon, chum salmon, sockeye salmon, pink salmon, masu salmon, trout species such as rainbow trout, brook trout, brown trout, common grayling, Arctic grayling, Arctic char, moronids, e.g. bass, striped bass, white bass, striped-white bass hybrids, yellow bass, perch such as white perch, yellow perch or European perch, bass-perch hybrids, cichlids, e.g. Nile tilapia, blue tilapia, blue-Nile tilapia hybrids, Mozambique tilapia, cyprinids, e.g. zebrafish and other carp species, breams, e.g. seabreams, porgies, gadids, e.g. cod, haddock, whiting, pollock and catfish species.

In one preferred embodiment, the fish is one for aquaculture such as a salmon, a trout, a bass, a tilapia, a carp or a catfish. In another preferred embodiment, the fish is one for aquarium trade, e.g. a zebrafish, a goldfish, a catfish, a tetra, a hatchet fish, a pencil fish, a goby, a damselfish, a clownfish, or chromis.

In another preferred embodiment, the fish is
a salmonid, moronid, cichlid, gadid, pangasid, ictalurid or cyprinid such as a salmon, a trout, a bass, a tilapia, a carp or a catfish.

As used herein, the term "gapmer" refers to a single-stranded, antisense oligonucleotide having a contiguous wing-gap-wing sequence. The gap comprises a continuous stretch of RNase H recruiting phosphorothioate-modified 2'-deoxynucleotides. The gap is flanked by a 5' wing region and a 3' wing region, wherein the nucleotides in said wing regions are locked nucleic acids (LNAs) or 2'-alkylated RNA nucleotides.

The gap of the gapmer may consist of 6 to 20 linked RNase H recruiting phosphorothioate-modified 2'-deoxynucleotides, such as 6 to 20, 6 to 18, 6 to 16, 6 to 14, 6 to 12 or 6 to 10 or 7 to 20, 7 to 18, 7 to 16, 7 to 14, 7 to 12, 7 to 10 or 8 to 20, 8 to 18, 8 to 16, 8 to 14, 8 to 12, 8 to 10 or 9 to 20, 9 to 18, 9 to 16, 9 to 14, 9 to 12 or 9 to 10 or 10 to 20, 10 to 18, 10 to 16, 10 to 14 or 10 to 12.

The 5' wing region of the gapmer may consist of 1 to 8 linked nucleotides, such as 1 nucleotide, 2 nucleotides, 3 nucleotides, 4 nucleotides, 5 nucleotides, 6 nucleotides, 7 nucleotides or 8 nucleotides, wherein said nucleotides are LNAs or 2'-alkylated RNA nucleotides.

The 3' wing region of the gapmer may consist of 1 to 8 linked nucleotides, such as 1 nucleotide, 2 nucleotides, 3 nucleotides, 4 nucleotides, 5 nucleotides, 6 nucleotides, 7 nucleotides or 8 nucleotides, wherein said nucleotides are LNAs or 2'-alkylated RNA nucleotides.

The 5' wing region may consist of the same number of linked LNAs or 2'-alkylated RNA nucleotides as the 3' wing region or the 5' wing region and the 3' wing region may consist of a different number of linked LNAs or 2'-alkylated RNA nucleotides.

Each LNA or 2'-alkylated RNA nucleotide of the wing region may be an affinity-enhancing chemically modified nucleotide. Examples of such nucleotides are LNA in the α-L-conformation or β-D-conformation, 2'-O-methyl-RNA nucleotides or 2'-O-methoxyethyl-RNA nucleotides.

The gapmer may consist of 8 to 36 nucleotides. In some embodiments, the gapmer consists of 10 to 22 nucleotides, such as 12 to 18, 13 to 17 or 12 to 16 nucleotides, e.g. 12, 13, 14, 15 or 16 nucleotides or 14, 15, 16, 17, 18, 19 or 20 nucleotides. In some embodiments, the gapmer consists of no more than 22 nucleotides, e.g. no more than 20 nucleotides or no more than 18 nucleotides, such as 15, 16 or 17 nucleotides. In some embodiments, the gapmer comprises less than 20 nucleotides.

The wing-gap-wing motif of the gapmer may be 5-8-5, 5-6-5, 4-10-4, 4-8-4, 46-4, 3-12-3, 3-10-3, 3-8-3, 2-16-2, 2-14-2, 2-12-2, 2-10-2, 1-16-1, 1-14-1, 1-12-1, 110-1, 2-8-2, 1-8-1, 3-6-3 or 1-6-1.

The internucleoside linkages between the nucleotides in the gapmer may be all phosphorothioate internucleoside linkages. In some such embodiments, the phosphorothioate internucleoside linkages are stereodefined, i.e. [all Rp] configuration or [all Sp] configuration. In other embodiments, the phosphorothioate internucleoside linkages are not stereodefined, i.e. racemic mixtures of Rp and Sp. In yet another embodiment, the phosphothioate internucleoside linkages within the 5' wing region and/or the gap and/or the 3' wing region are stereodefined.

Gapmers can be synthesized by methods known in the art. They are commercially available, e.g. from Qiagen, Bio-Synthesis Inc., Exiqon and the like.

The sequence of the gapmer can be designed using available gene sequence database and bioinformatics tools to achieve high target affinity, sequence specificity and biological stability. Companies offering custom gapmer synthesis often also offer tools for their design.

As used herein, the term "sterile" fish refers to an individual that is unable to sexually reproduce. Sterile fish are defined as individuals that are unable to reach sexual maturity or to reproduce when reaching the age of sexual maturity.

The production of sterile fish according to the invention involves the disruption of gonadal development in the embryo developed from the eggs of such animals after fertilization. This is achieved by transfecting the eggs with a gapmer that has a sequence suitable for targeting RNA of a gene selected from *dead end (dnd), nanos, vasa, piwi, gnrh* or *fsh* receptor, i.e. which is capable of the disruption of gonadotropin releasing hormone (GnRH) cell development and/or primordial germ cell (PGC) development by knockdown of mRNA of said genes and thus disrupting the generation of a gene product responsible for or involved in GnRH cell and/or PGC development, migration and/or colonization.

The gapmer used in the method of the invention catalyzes RNase H-dependent degradation of complementary mRNA targets, thus "knocking down" such targets: the wing regions of the gapmer achieve sufficient target binding affinity and confer nuclease resistance. When the gapmer is hybridized to its target RNA, the central gap activates RNase H cleavage of the opposing RNA strand and thus the degradation of the target RNA. The gapmer for use in the method of the invention thus has a sequence which is suitable to target mRNA of a gene which is essential for embryonic germ cell development selected from *dnd, nanos, vasa, piwi, gnrh* or *fsh* receptor, cleave said RNA and suppress the expression of the respective protein, that is essential for embryonic germ cell development, thereby causing failure of fertile gonadal development.

The gapmer for use in the method of the invention has a sequence suitable for targeting RNA of genes selected from *dnd, nanos, vasa, piwi, gnrh* or *fsh* receptor that are essential for embryonic germ cell development. The specific sequence of the gapmer is dependent on the target RNA, the location of binding within the target RNA and the species of fish. In a preferred embodiment, the gapmer for use in the method of the invention has a sequence suitable for targeting *dnd* RNA*.* Knockdown of said genes results in the failure of fertile gonadal development and ultimately generates sterile egg-producing aquatic animals.

The term "targeting" as used herein refers to the gapmer binding to/hybridizing to a complementary RNA.

The term "egg" as used herein refers to the vessel containing a zygote or an embryo, i.e. a fertilized egg.

The eggs transfected by the method of the invention are pre-water activated fertilized eggs. In such eggs, the structure of the chorion has changed such that the passage of molecules is limited.

The eggs are transfected by immersion in an aqueous immersion medium. Generally, the immersion medium comprises at least the gapmer and water. In one embodiment, the aqueous immersion medium consists of the gapmer and water. In another embodiment, the immersion medium may further comprise other compounds, including compounds that assist or promote the transfection and/or compounds that are beneficial to the embryos/fish hatched from the transfected eggs.

Compounds which assist or promote the transfection as described in the claims include salts such as acetates, citrates, borates, phosphates and the like, buffering agents which keep the pH of the aqueous immersion medium within a desired range, preferably in the range of 7-9, including Tris, HEPES, MOPS and the like and chelating agents such as EDTA, amino acids like glutamine, glycine and the like.

Compounds that are beneficial to the embryos/fish hatched from the transfected eggs include hormones, growth promoters, protective antigens, antibiotics, nutrients and the like.

Other compounds which may be present in the aqueous immersion medium include culture medium for eggs of fish, ovarian fluid, serum, protease inhibitors and the like.

The eggs are immersed in the aqueous immersion medium and the gapmer is naturally taken up by the eggs. Such unassisted uptake is also known as gymnosis. The immersed eggs may be briefly sonicated. Gapmer concentrations may be in the range of 1 nM to 250 µM, e.g. 1 nM to 5 nM or 5 nM to 50 nM or 10 nM to 20 nM or 100 nM to 250 µM, e.g. 100 to 250 nM, 250 to 500 nM, 500 to 1000 nM, 1µM to 50 µM, 50µM to 100 µM, 100 µM to 150 µM, 150 µM to 200 µM or 200 µM to 250 µM, such as at least 5 nM, at least 10 nM, at least 20 nM, at least 50 nM, at least 100 nM, at least 250 nM, at least 500 nM, at least 1µM, at least 50 µM, at least 100 µm, at least 150 µM, at least 200 µM.

The time required for satisfactory transfection, i.e. time required for the eggs to be immersed in the immersion medium will depend on the type of egg (fish species).

If transfection is carried out by gymnosis as claimed, the eggs may be immersed in the aqueous immersion medium comprising the gapmer for 1 to 144 hours, e.g. 1 hour to 2 hours, 2 hours to 6 hours, 6 hours to 12 hours, 12 hours to 24 hours, 24 hours to 36 hours, 36 hours to 48 hours, 48 hours to 120 hours or 4 hours to 12 hours, 24 hours to 96 hours, or 36 to 72 hours, such as at least 2 hours, at least 4 hours, at least 6 hours, at least 8 hours, at least12 hours, at least 24 hours, at least 36 hours, at least 48 hours, at least 60 hours, at least 72 hours, at least 84 hours, at least 96 hours, at least 108 hours, at least 120 hours or at least 132 hours. For long immersion periods, it may be beneficial to replace the immersion medium with fresh immersion medium.

The temperature of the aqueous immersion medium depends on the type of egg/fish species and the duration of the transfection, i.e. the time the eggs have to be kept in the aqueous immersion medium for them to be transfected. For gymnosis as claimed, the temperature of the aqueous immersion medium will typically be kept in the range of the water temperature of the eggs' natural habitat. For eggs of cold water fish such as salmon, the temperature of the immersion medium is typically kept to 2 to 8 °C, e.g. 2 to 4°C, 4 to 6°C or 3 to 8°C, while for eggs of warm water fish such as tilapia, the temperature of the immersion medium is typically kept to 21 to 25 °C.

The success of the transfection, i.e. the degradation of the target RNA and suppression of protein expression, can easily be determined by quantitative PCR (qPCR); hence, successfully transfected eggs can be identified and selected and there is no need to follow the embryos until maturation of the gonads to show that GnRH cell and/or PGC development, migration and/or colonization was disrupted and resulting adult egg-producing aquatic animals will be reproductively sterile.

After transfection of pre-water activated fertilized eggs, such eggs are water-activated. Prior to water activation the eggs may be washed with water or a suitable medium before being transferred into a habitat/an environment, which allows the embryo to develop, grow and ultimately the fish to hatch. After transfection of fertilized eggs, such eggs may be washed with water or a suitable medium before being transferred into a habitat/an environment, which allows the embryo to develop, grow and ultimately the fish to hatch.

The method of the invention is useful to produce reproductively sterile fish. Sterilization (induced infertility) of farmed fish enhances their growth rate by increasing the conversion of food energy to muscle growth, instead of gonadal development. In addition, if escaped from aquaculture operations to the environment, sterile fish, including domesticated, non-native or genetically modified species, will not be able to reproduce or inter-breed with wild stock. This will assist biological containment and prevent genetic contamination of wild populations and/or the establishment in the wild of domestic, non-native or genetically modified farmed fish.

In one embodiment, the gapmer for use in the method of the invention has a sequence suitable for targeting *dnd* RNA in Atlantic salmon, e.g. SEQ ID NO: 1, SEQ ID NO: 2 or SEQ ID NO: 3 or a variant thereof, which differs from these sequences by 1 to 5 nucleotides and which is suitable for targeting *dnd* RNA in Atlantic salmon. In another embodiment, the gapmer for use in the method of the invention consists of a gapmer of SEQ ID NO: 1 and a gapmer of SEQ ID NO: 2 (or variants thereof, which differ from these sequences by 1 to 5 nucleotides and which are suitable for targeting *dnd* RNA in Atlantic salmon) or a gapmer of SEQ ID NO: 1 and a gapmer of SEQ ID NO: 3 (or variants thereof, which differ from these sequences by 1 to 5 nucleotides and which is suitable for targeting *dnd* RNA in Atlantic salmon) or a gapmer of SEQ ID NO: 2 and a gapmer SEQ ID NO: 3 (or variants thereof, , which differ from these sequences by 1 to 5 nucleotides and which is suitable for targeting *dnd* RNA in Atlantic salmon) or a gapmer of SEQ ID NO: 1, a gapmer of SEQ ID NO: 2 and a gapmer of SEQ ID NO: 3 (or variants thereof, which differ from these sequences by 1 to 5 nucleotides and which is suitable for targeting *dnd* RNA in Atlantic salmon).

The invention will be illustrated by the following examples.

### Examples

### Example 1: Transfection of fertilized Salmo salar eggs by immersion in an aqueous immersion medium comprising a gapmer that targets dead end mRNA

Gapmers 1-3 that target Salmo salar *dead end (dnd)* mRNA and gapmer 4 having a non-*dnd* specific sequence (negative control) were designed using the online Qiagen gapmer design tool and purchased from Qiagen:
Gapmer 1: TTGAACGCTCCTCCAT (SEQ ID NO: 1)
Gapmer 2: GGAGCAGCGAGGAGGT (SEQ ID NO: 2)
Gapmer 3: GCAAAACATTTAAGTA (SEQ ID NO: 3)
Gapmer 4: GCTCCCTTCAATCCAA (SEQ ID NO: 4)

The gap of the gapmers comprised phosphorothioate-modified nucleotides while the wings comprised LNAs.

Salmo salar eggs were fertilized in an aqueous immersion medium (150 mM NaCl, 3.3 mM KCl, 20 mM Tris-HCl, pH 8.4) for 10 minutes before the fertilized eggs were collected and rinsed once in the aqueous immersion medium. The eggs were placed into 10 test tubes (3 eggs/test tube) and immersed in an aqueous immersion medium (150 mM NaCl, 3.3 mM KCl, 20 mM Tris-HCl, pH 8.4) containing either 10 nM of gapmer 1, 2, 3 or 4 (2 test tubes each) or no gapmer (2 test tubes). Of the two equal test tubes, one was briefly sonicated (10 s, 42 kHz). All test tubes were stored at 8°C in the dark for 4 hours, then RNA was extracted according to the TRIzol method, and treated with DNase to completely remove any DNA from the extracted RNA.

RNAs were reverse transcribed into cDNAs using the Qiagen OneStep RT-PCR kit, following the manufacturer's instructions. PCR was carried out with a BioRad MyCycler thermocycler using the OneTaq Polymerase kit (New England Biolabs) and following the manufacturer's instructions. The following *dnd*-specific primers were used (amplicon size 401 bp):
Salgap2-F: GAGCGTTCAAGTCAGGTGTTG (SEQ ID NO: 5)
Salgap2-R: CAGAGCTGACGTTTCTCCGT (SEQ ID NO: 6)

PCR amplification of a common Salmo salar house-keeping gene was carried out to control integrity of the extracted RNA/transcribed cDNA. All samples showed an equally strong band for the house-keeping gene. No-template PCR controls were blank, i.e. showing that there was no *dnd* template contamination in the PCR reagents and/or the equipment which could produce false results.

Eggs which had been immersed in immersion medium only (without gapmer) gave strong PCR signals, showing that the *dnd*-mRNA was detectable in such eggs. Eggs which had been immersed in immersion medium comprising gapmer 4 also gave strong PCR signals, showing that the random gapmer sequence did not target *dnd* mRNA.

Eggs which had been immersed in immersion medium comprising gapmer 1 gave very weak (with sonication) and no PCR signal (without sonication), showing that the eggs had been successfully transfected and that gapmer 1 was effective to target and destroy *dnd*-mRNA, i.e. knockdown of the *dead end* gene which is crucial for the migration and survival of PGCs. If the eggs had been allowed to develop, the fish hatched therefrom would have been reproductively sterile.

Eggs which had been immersed in immersion medium comprising gapmer 2 gave a weak PCR signal (with or without sonication), showing that the eggs had been successfully transfected and that gapmer 2 was to a large degree effective to target and destroy *dnd*-mRNA. It is likely that gapmer 2 could show equally good effectivity under optimized incubation conditions, e.g. longer incubation time.

Eggs which had been immersed in immersion medium comprising gapmer 3 gave a strong PCR signal (with sonication) and no PCR signal (without sonication). It is believed that the strong PCR signal in the sonicated eggs is not due to failure to transfect the eggs or lack of effectiveness of the gapmer but rather due to the eggs having suffered from the mechanical treatment. This hypothesis is supported by the absence of the PCR signal in the non-sonicated eggs which shows that the eggs had been successfully transfected and that gapmer 3 was effective to target and destroy *dnd*-mRNA, i.e. knockdown of the *dead end* gene which is crucial for the migration and survival of PGCs. If the eggs had been allowed to develop, the fish hatched therefrom would have been reproductively sterile.

The following Examples 2 and 3 are not according to the invention and are present for illustration purposes only.

### Example 2: Transfection of fertilized Salmo salar eggs by microinjection of a gapmer that targets dead end mRNA

Salmo salar eggs are fertilized in water supplemented with glutathione to prevent hardening of the chorion. At the one-cell stage, eggs are collected and divided up in 5 batches (10 eggs/batch) and the gapmers described in Example 1 are dissolved in physiological saline to prepare solutions of gapmers 1-4 at a concentration of 400 ng/µl. One batch of eggs is microinjected with a gapmer solution, i.e. batch 1/solution of gapmer 1, batch 2/solution of gapmer 2 and so on while batch 5 is injected with physiological saline only (same volume as gapmer solution). Microinjection is carried out using a World Precision instruments, PV820 pneumatic PicoPump, coupled with a Narishige MN-151 micromanipulator. After injection, the eggs are cultured in water containing glutathione at 8°C in the dark for 24 hours. From each batch, 3 eggs are transferred into a test tube for RNA extraction, reverse transcription and PCR which is carried out as described in Example 1. The absence of or a weak PCR signal shows that the transfection is successful and that the gapmer is effective to target and destroy *dnd*-mRNA, i.e. knockdown of the *dead end* gene which is crucial for the migration and survival of PGCs. The remaining eggs are rinsed in physiological saline and transferred for further development into their natural habitat until reproductively sterile fish hatch therefrom.

### Example 3: Transfection of fertilized Salmo salar eggs by immersion in an aqueous immersion medium comprising a gapmer that targets dead end mRNA and a transfection agent

Salmo salar eggs are fertilized in an aqueous immersion medium (150 mM NaCl, 3.3 mM KCl, 20 mM Tris-HCl, pH 8.4) for 10 minutes before the fertilized eggs are collected and rinsed once in the same aqueous immersion medium. The eggs are placed into 10 test tubes (10 eggs/test tube) and immersed in an aqueous immersion medium (150 mM NaCl, 3.3 mM KCl, 20 mM Tris-HCl, pH 8.4) containing a pre-mix of the gapmer 1, 2, 3 or 4 and Oligofectamine^{™} (Thermofisher) following the manufacturer's instructions or only the transfection agent and no gapmer. The concentration of gapmer in the gapmer-containing immersion media is 8 nM. All test tubes are stored at 8°C in the dark for 6 hours. From each test tube, 3 eggs are transferred into a fresh test tube for RNA extraction, reverse transcription and PCR which is carried out as described in Example 1. The absence of or a weak PCR signal shows that the transfection is successful and that the gapmer is effective to target and destroy *dnd*-mRNA, i.e. knockdown the of *dead end* gene which is crucial for the migration and survival of PGCs. The remaining eggs are rinsed in physiological saline and transferred for further development into their natural habitat until reproductively sterile fish hatch therefrom.

## Claims

1. A method for producing sterile fish, said method comprising transfecting fertilized fish eggs with a gapmer that is effective to render individuals produced therefrom sterile by immersion in an aqueous immersion medium that comprises said gapmer, wherein the gapmer has a sequence suitable for targeting RNA of a gene selected from *dead end (dnd), nanos, vasa, piwi, gnrh* or *fsh* receptor, wherein the gapmer comprises a gap that comprises a continuous stretch of RNase H recruiting phosphorothioate-modified 2'-deoxynucleotides and wherein said gap is flanked by a 5' wing region and a 3' wing region, wherein the nucleotides in said wing regions are locked nucleic acids (LNAs) or 2'-alkylated RNA nucleotides, and wherein the gapmer is taken up into the eggs by gymnosis.

2. The method according to claim 1, wherein the gapmer has a sequence suitable for targeting *dnd* RNA.

3. The method according to any of the preceding claims, wherein the gapmer consists of 8 to 36 nucleotides, preferably 10 to 22 nucleotides and more preferably, 14 to 20 nucleotides.

4. The method according to any of the preceding claims, wherein the fish is a salmonid, moronid, cichlid, gadid, pangasid, ictalurid or cyprinid.

5. The method according to claim 4, wherein the fish is a salmonid.

6. The method according to claim 5, wherein said salmonid is Atlantic salmon, coho salmon, chinook salmon, chum salmon, sockeye salmon, pink salmon, masu salmon, rainbow trout, brook trout, brown trout, common grayling, Arctic grayling or Arctic char.

7. The method according to claim 6, wherein said salmonid is Atlantic salmon.

8. The method according to claim 7, wherein the gapmer has a sequence selected from SEQ ID NO: 1, SEQ ID NO: 2 or SEQ ID NO: 3 or a variant thereof, which differs from these sequences by 1 to 5 nucleotides and which is suitable for targeting *dnd* RNA in Atlantic salmon.

9. The method according to claim 8, wherein said gapmer has a sequence selected from SEQ ID NO: 1, SEQ ID NO: 2 or SEQ ID NO: 3.

10. The method according to any of the preceding claims, wherein the nucleotides in said wing regions are LNAs.

## Patentansprüche

1. Verfahren zur Erzeugung steriler Fische, wobei das Verfahren die Transfektion von befruchteten Fischeiern mit einem Gapmer umfasst, das wirksam ist, um daraus erzeugte Individuen durch Eintauchen in ein wässriges Immersionsmedium, das das Gapmer umfasst, steril zu machen, wobei das Gapmer eine Sequenz umfasst, die für das Targeting von RNA eines Gens geeignet ist, das aus dead end (dnd), nanos, vasa, piwi, gnrh oder fsh-Rezeptor ausgewählt ist, wobei das Gapmer eine Lücke umfasst, die eine kontinuierliche Strecke von RNase H rekrutierenden phosphorothioat-modifizierten 2'-Desoxynukleotiden umfasst, und wobei die Lücke von einer 5'-Flügelregion und einer 3'-Flügelregion flankiert ist, wobei die Nukleotide in den Flügelregionen verbrückte Nukleinsäuren (LNAs) oder 2'-alkylierte RNA-Nukleotide sind, und wobei das Gapmer durch Gymnosis in die Eier aufgenommen wird.

2. Verfahren nach Anspruch 1, wobei das Gapmer eine Sequenz aufweist, die für das Targeting von dnd-RNA geeignet ist.

3. Verfahren nach einem der vorhergehenden Ansprüche, wobei das Gapmer aus 8 bis 36 Nukleotiden, vorzugsweise aus 10 bis 22 Nukleotiden und noch bevorzugter aus 14 bis 20 Nukleotiden besteht.

4. Verfahren nach einem der vorhergehenden Ansprüche, wobei der Fisch ein Salmonide, Moronide, Cichlid, Gadide, Pangaside, Iktaluride oder Cyprinide ist.

5. Verfahren nach Anspruch 4, wobei der Fisch ein Salmonide ist.

6. Verfahren nach Anspruch 5, wobei es sich bei dem Salmonide um Atlantischen Lachs, Coho-Lachs, Königslachs, Ketalachs, Rotlachs, Buckellachs, Masu-Lachs, Regenbogenforelle, Bachsaibling, Forelle, Äsche, Arktische Äsche oder Seesaibling handelt.

7. Verfahren nach Anspruch 6, wobei es sich bei dem Salmoniden um Atlantischen Lachs handelt.

8. Verfahren nach Anspruch 7, wobei das Gapmer eine Sequenz aufweist, ausgewählt aus SEQ ID NO: 1, SEQ ID NO: 2 oder SEQ ID NO: 3 oder einer Variante davon, die sich von diesen Sequenzen um 1 bis 5 Nukleotide unterscheidet und die zum Targeting von dnd-RNA in Atlantischem Lachs geeignet ist.

9. Verfahren nach Anspruch 8, wobei das Gapmer eine Sequenz aufweist, ausgewählt aus SEQ ID NO: 1, SEQ ID NO: 2 oder SEQ ID NO: 3.

10. Verfahren nach einem der vorhergehenden Ansprüche, wobei die Nukleotide in den Flügelregionen LNAs sind.

## Revendications

1. Procédé de production de poissons stériles, ledit procédé comprenant la transfection d'oeufs de poisson fécondés avec un gapmère qui est efficace pour rendre stériles les individus produits à partir de ceux-ci par immersion dans un milieu d'immersion aqueux qui comprend ledit gapmère, dans lequel le gapmère présente une séquence appropriée pour cibler l'ARN d'un gène sélectionné parmi les récepteurs *Dead End (dnd)*, *nanos, vasa, piwi, gnrh* ou *fsh,* dans lequel le gapmère comprend un espace qui comprend une étendue continue de RNase H recrutant des 2'-désoxynucléotides modifiés par un phosphorothioate et dans lequel ledit espace est flanqué d'une région d'aile 5' et d'une région d'aile 3', dans lequel les nucléotides dans lesdites régions d'aile sont des acides nucléiques verrouillés (LNA) ou des nucléotides d'ARN 2'-alkylés, et dans lequel le gapmère est absorbé dans les oeufs par gymnose.

2. Procédé selon la revendication 1, dans lequel le gapmère présente une séquence adaptée au ciblage de l'*ARN* dnd.

3. Procédé selon l'une quelconque des revendications précédentes, dans lequel le gapmère est constitué de 8 à 36 nucléotides, de préférence de 10 à 22 nucléotides et de manière davantage préférée de 14 à 20 nucléotides.

4. Procédé selon l'une quelconque des revendications précédentes, dans lequel le poisson est un salmonidé, un moronidé, un cichlidé, un gadidé, un pangasidé, un ictaluridé ou un cyprinidé.

5. Procédé selon la revendication 4, dans lequel le poisson est un salmonidé.

6. Procédé selon la revendication 5, dans lequel ledit salmonidé est le saumon atlantique, le saumon argenté, le saumon quinnat, le saumon kéta, le saumon rouge, le saumon rose, le saumon masou, la truite arc-en-ciel, l'omble de fontaine, la truite brune, l'ombre gris, l'ombre arctique ou l'omble chevalier.

7. Procédé selon la revendication 6, dans lequel ledit salmonidé est le saumon atlantique.

8. Procédé selon la revendication 7, dans lequel le gapmère présente une séquence choisie parmi SEQ ID n° 1, SEQ ID n° 2 ou SEQ ID n° 3 ou une variante de celles-ci, qui diffère de ces séquences par 1 à 5 nucléotides et qui est adaptée pour cibler l'ARN *dnd* chez le saumon atlantique.

9. Procédé selon la revendication 8, dans lequel ledit gapmère présente une séquence choisie parmi SEQ ID n° 1, SEQ ID n° 2 ou SEQ ID n° 3.

10. Procédé selon l'une quelconque des revendications précédentes, dans lequel les nucléotides dans lesdites régions d'ailes sont des LNA.
